(19) Europäisches Patentamt
European Patent Office
Office européen des brevets

(11) Publication number: **0 397 859 A1**

(12) # EUROPEAN PATENT APPLICATION published in accordance with Art. 158(3) EPC

(21) Application number: 88909824.0

(22) Date of filing: 11.11.88

(86) International application number: PCT/JP88/01141

(87) International publication number: WO 89/04308 (18.05.89 89/11)

(51) Int. Cl.$^5$: C07D 241/12, C07D 241/16, C07D 241/24, C07D 241/42, A61K 31/495

(30) Priority: 12.11.87 JP 286197/87
12.11.87 JP 286198/87
20.11.87 JP 293423/87

(43) Date of publication of application: 22.11.90 Bulletin 90/47

(84) Designated Contracting States: BE CH DE FR GB IT LI NL SE

(71) Applicant: Terumo Kabushiki Kaisha
No. 44-1, Hatagaya 2-chome Shibuya-ku
Tokyo 151(JP)

(72) Inventor: SUWABE, Yasushi Terumo Kabushiki Kaisha
44-1, Hatagaya 2-chome Shibuya-ku
Tokyo 151(JP)
Inventor: USHIJIMA, Hideto Terumo Kabushiki Kaisha
44-1, Hatagaya 2-chome
Shibuya-ku Tokyo 151(JP)
Inventor: HIJIKURO, Kohshi Terumo Kabushiki Kaisha
44-1, Hatagaya 2-chome
Shibuya-ku Tokyo 151(JP)
Inventor: SAKURAGI, Shiho Terumo Kabushiki Kaisha
44-1, Hatagaya 2-chome
Shibuya-ku Tokyo 151(JP)
Inventor: SUZUKI, Tadahiko Terumo Kabushiki Kaisha
44-1, Hatagaya 2-chome Shibuya-ku
Tokyo 151(JP)
Inventor: AKITA, Yasuo
28-7, Akebonocho 3-chome
Tachikawa-shi Tokyo 190(JP)
Inventor: OHTA, Akihiro
10-8, Nishikoiwa 3-chome Edogawa-ku
Tokyo 133(JP)

(74) Representative: Gillard, Marie-Louise et al
Cabinet Beau de Loménie 55, Rue d'Amsterdam
F-75008 Paris(FR)



Xerox Copy Centre

(54) **PYRAZINE DERIVATIVES AND MEDICINAL PREPARATION CONTAINING SAME.**

(57)

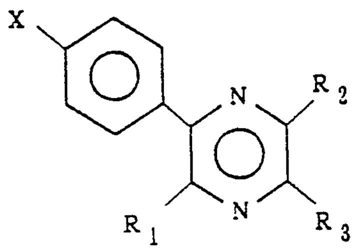

(I)

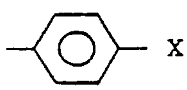

(a)

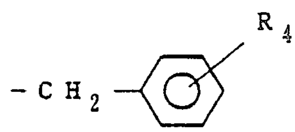

(b)

Pyrazine derivatives represented by general formula (I), wherein X represents a hydrogen atom, a halogen atom, a lower alkyl group, a lower alkoxy group or a cyano group, $R_1$ represents a hydrogen atom, a lower alkyl group or a group of (a) (wherein X is as defined above), $R_2$ represents a hydrogen atom, a halogen atom or (a) (wherein X is as defined above), $R_3$ represents a hydrogen atom, a halogen atom, a lower alkyl group, a cyano group, a naphthylmethyl group, a benzyl group or (b) (wherein $R_4$represents a hydrogen atom, a halogen atom or a lower alkylamino group), a carboxyl group or a lower alkoxycarbonyl group or, when taken together, $R_2$ and $R_3$ represent a cyclohexane ring or a benzene ring together with the carbon atoms to which they are bound, provided that the case where X represents a hydrogen atom, $R_1$ represents (a) (wherein X represents a hydrogen atom), and $R_2$ and $R_3$ represent hydrogen atoms and the case where X represents a hydrogen atom, $R_1$ represents a hydrogen atom, $R_2$ represents (a) (wherein X represents a hydrogen atom), and $R_3$ represents a methyl group are excluded. The pyrazine derivatives have a strong blood platelet agglutination depressing effect and a cyclooxygenase inhibiting effect. The above-described compounds are used as blood platelet agglutination inhibitor or anti-inflammatory agents.

TITLE MODIFIED
see front page

# SPECIFICATION

## Pyrazine derivatives and pharmaceutical preparations containing the same

### Technical Field

This invention relates to novel pyrazine derivatives and pharmaceutical preparations containing the same.

The pyrazine derivative of the invention possess a potent platelet aggregation-inhibiting activity and are effective for preventing diseases caused by aggregation of the platelets such as thrombosis. As compounds having a cyclooxygenase-inhibiting activity are known, in general, to possess an antiinflammatory activity, the pyrazine derivatives of the invention having such inhibitory action find use as an antiinflammatory agent.

### Technological Background

There have been known various substances which have platelet aggregation-inhibiting activities. Their activities, however, are so weak that development of improved agents is desired. There is also strong need for antithrombotic agents that will effectively prevent thromboses such as myocardial infarction and cerebral thrombosis which have recently become the majority of adult diseases.

On the other hand, a variety of pyrazine derivatives have recently been known such as, for example, 2,3-diphenylpyrazine described in Journal of Heterocyclic Chemistry, vol. 21, pp. 103-106. However, none of these pyrazine derivatives are known to possess a platelet aggregation-inhibiting activity.

As a result of extensive studies on pharmacological activities of novel pyrazine derivatives prepared by us, we have found that certain pyrazine derivatives possess excellent platelet aggregation-inhibitory and antiinflammatory activities and have completed the present invention.

Disclosure of the Invention

According to the present invention there are provided pyrazine derivatives represented by the general formula (I)

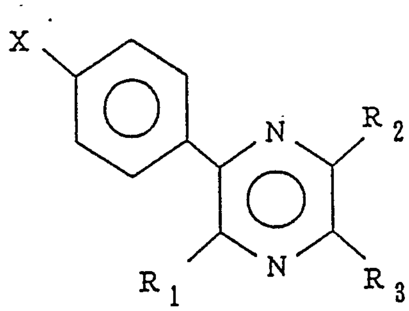

(I)

wherein X represents a hydrogen atom, a halogen atom, a lower alkyl group, a lower alkoxy group or a cyano group; $R_1$ represents a hydrogen atom, a lower alkyl group or a group having the formula 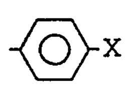X in which X has the same meaning as

defined above; $R_2$ represents a hydrogen atom, a halogen atom or a group having the formula 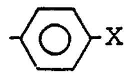-X in which X has the same meaning as defined above; $R_3$ represents a hydrogen atom, a halogen atom, a lower alkyl group, a cyano group, a naphthylmethyl group, a benzyl group having the formula $-CH_2-$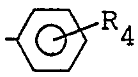 in which $R_4$ represents a hydrogen atom, a halogen atom, or a lower alkylamino group, a carboxyl group or a lower alkoxycarbonyl group, or $R_2$ and $R_3$ together with the carbon atoms to which they are connected represent a cyclohexane ring or a benzene ring except for the case where X is a hydrogen atom, $R_1$ is a group having the formula 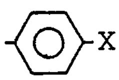-X in which X is a hydrogen atom and $R_2$ and $R_3$ independently are a hydrogen atom or X is a hydrogen atom, $R_1$ is a hydrogen atom, $R_2$ is a group having the formula 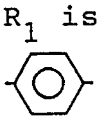-X in which X is a hydrogen atom and $R_3$ is a methyl group, and platelet aggregation-inhibiting agents and antiinflammatory agents containing said pyrazine derivatives.

Further according to the invention there are provided a method for preventing thrombosis and a method for treating inflammation which comprise administering an effective amount of the above-mentioned pyrazine derivatives.

In the definition of the substituents X, $R_1$, $R_2$ and $R_3$ in the above formula (I), the lower alkyl group includes, for example, methyl, ethyl, propyl, isopropyl,

butyl and isobutyl, the halogen atom includes, for example, fluorine, chlorine and bromine, the lower alkoxy group includes, for example, methoxy, ethoxy, propoxy, isopropoxy, butoxy and isobutoxy, the lower alkyloxycarbonyl group includes, for example, methoxycarbonyl, ethoxycarbonyl, propoxycarbonyl, isopropoxycarbonyl, butoxycarbonyl and isobutoxycarbonyl and the lower alkylamino group includes, for example, methylamino, ethylamino, propylamino, isopropylamino, butylamino and isobutylamino.

The compounds (I) in the present invention are preferably pyrazine derivatives having the formula:

1) pyrazine derivatives represented by the general formula (II)

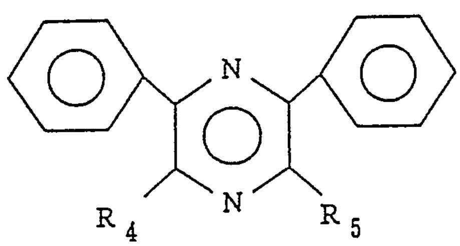

(II)

wherein $R_4$ and $R_5$ which may be the same or different independently represent a hydrogen atom or a lower alkyl group except for the case where $R_4$ represents a hydrogen atom and $R_5$ represents a methyl group;

2) pyrazine derivatives represented by the general formula (III) or (IV)

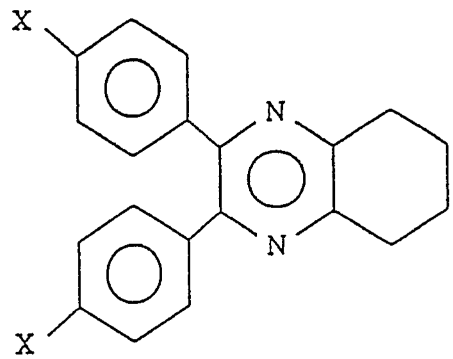

(III)

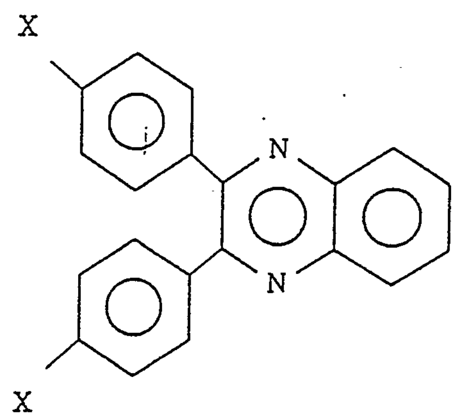

(IV)

wherein X represents a hydrogen atom, a halogen atom, a lower alkyl group, a lower alkoxy group or a cyano group, or

3) pyrazine derivatives represented by the general formula (V)

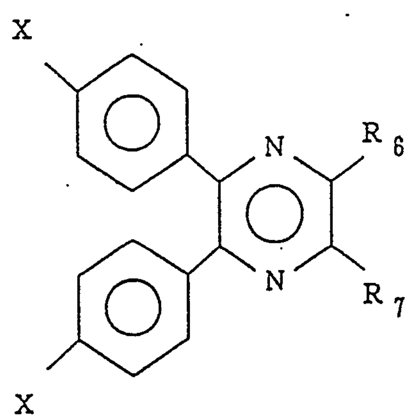

(V)

wherein X represents a hydrogen atom, a cyano group or a lower alkoxy group, $R_6$ represents a hydrogen atom or a halogen atom and $R_7$ represents a hydrogen atom, a halogen atom, a lower alkyl group, a cyano group, a naphthylmethyl group, a benzyl group which may contain as the substituent a halogen atom or a lower alkylamino group, a carboxyl group or a lower alkyloxycarbonyl group.

The pyrazine derivatives of the invention having the above formula (I) are prepared depending upon the nature of $R_1$, $R_2$ and $R_3$ as follows:

1) A pyrazine derivative of the formula (I) wherein $R_1$ and $R_3$ are independently a lower alkyl group and $R_2$ is a group having the formula 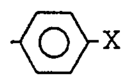X is prepared by reacting a 2,6-dichloro-3,5-diphenylpyrazine derivative having the general formula (VI)

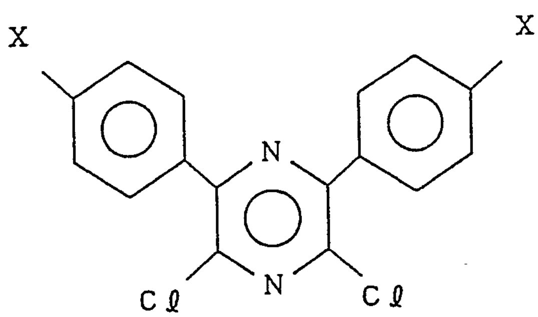

(VI)

wherein X has the same meaning as defined above with a tri-lower alkylboron;

2) a pyrazine derivative of the formula (I) wherein

$R_1$ is a group having the formula 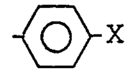 and $R_2$ and $R_3$ together with the carbon atoms to which they are connected form a cyclohexane ring or a benzene ring is prepared by heating a benzyl derivative having the general formula (VII)

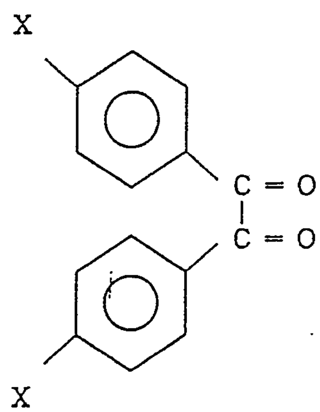

(VII)

wherein X has the same meaning as defined above and 1,2-diaminocyclohexane (VIII) or o-phenylenediamine (IX) in an appropriate organic solvent followed by heating with sulfur at 100-180°C

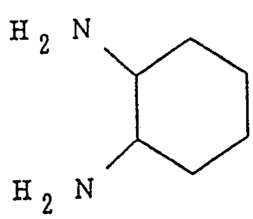

(VIII)

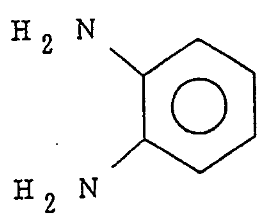

(IX)

3) a compound of the formula (I) wherein $R_1$ is a group having the formula 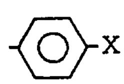 and both $R_2$ and $R_3$ are a hydrogen atom is prepared by heating a benzyl derivative

having the above-mentioned general formula (VII) and a 1,2-diamine having the formula (X)

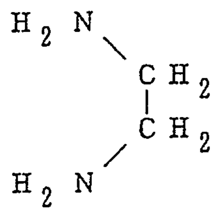

(X)

in an appropriate organic solvent, for example, ethanol to give a dihydropyrazine derivative having the general formula (XI)

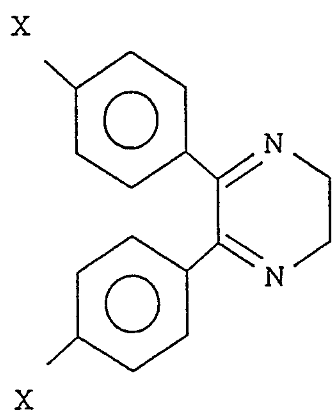

(XI)

wherein X has the same meaning as defined above and subsequently heating it with sulfur at 100-180°C;

4) a compound of the formula (I) wherein $R_1$ is a group having the formula 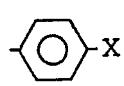-X, $R_2$ is a hydrogen atom and $R_3$ is a naphthylmethyl group or a benzyl group which may be

substituted is prepared by reacting the above-mentioned dihydropyrazine derivative (XI) with naphthaldehyde or a benzaldehyde which may have as the substituent a halogen atom or a lower alkylamino group;

5) a compound of the formula (I) wherein $R_1$ is a group having the formula 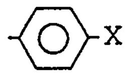X, $R_2$ is a hydrogen atom or a halogen atom and $R_3$ is a halogen atom is prepared by oxidizing the pyrazine derivative (I) wherein $R_2$ and $R_3$ are independently a hydrogen atom with permaleic acid to give a mono- or di-N-oxide, and then halogenating it with a phosphorus oxyhalide; and

6) a compound of the formula (I) wherein $R_1$ is a group having the formula 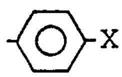X, $R_2$ is a hydrogen atom and $R_3$ is a cyano group is prepared by reacting a compound having the general formula (XII)

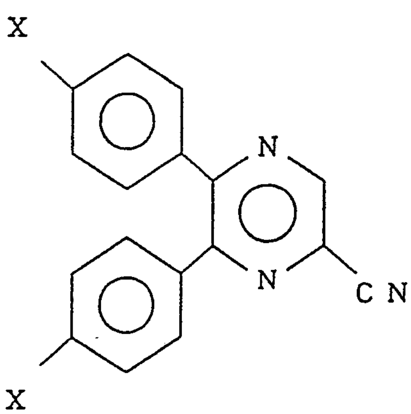

(XII)

wherein X has the same meaning as defined above with potassium cyanide in the presence of a palladium catalyst

such as tetrakis(triphenylphosphine)palladium.

Hydrolysis of said compound under alkaline conditions yields the compound (I) wherein $R_3$ is a carboxyl group. Further alkylation of the same affords the compound (I) wherein $R_3$ is a low alkyloxycarbonyl group.

As the pyrazine derivatives of the invention possess a platelet aggregation-inhibiting activity, they are effectively utilized as a platelet aggregation inhibition for the prevention of cerebral thrombosis and like diseases. In addition, the pyrazine derivatives of the invention possess a cyclooxygenase-inhibiting activity and are also utilizable as an antiinflammatory agent. They may be administered at a dose between about 30-600 mg per day in adults, if necessary, divided into one to three doses. Any suitable route for administration may be chosen, oral administration being particularly desirable. Intravenous administration is also acceptable.

The compounds of the invention are formulated either alone or in admixture with pharmaceutical carriers or excipients by a conventional method into tablet, powder, capsule or granule. As examples of the carrier or excipient are mentioned calcium carbonate, calcium phosphate, starch, sucrose, lactose, talc, magnesium stearate and the like. In addition to the above-mentioned solid preparations, the compounds of the invention may also be formulated into liquid preparations such as oily suspension or syrup.

The compounds of the invention may also be stabilized in the form of inclusion in cyclodextrin.

The invention will more concretely be illustrated below by means of examples and pharmacological test examples.

Example 1

To a solution of 2,6-dichloro-3,5-diphenylpyrazine (840 mg, 3 mM), anhydrous potassium carbonate (1.24 g, 9 mM) and tetrakis(triphenylphosphine)palladium (348 mg, 0.3 mM) in anhydrous DMF (15 ml) was added a 15% $Et_3B$-hexane solution* (6 ml, 6 mM) in small portions at room temperature. The mixture was heated under reflux under argon stream for 12 hours. The solvent was distilled off under reduced pressure followed by addition of water (20 ml). The mixture was extracted with methylene chloride, and the solvent is distilled off. There was obtained a brown viscous material, which was then subjected to medium pressure column chromatography on silica gel** (Column: Kieselgel 60, 230-400 mesh, manufactured by Merck, 20 mm x 200 mm; Solvent: Hexane-AcOEt=7:1). There was produced 50 mg of 2,6-diethyl-3,5-diphenylpyrazine (59%) as the first eluate.

* The solution manufactured by Kanto Kagaku K.K. used intact.

** UVILOG, ALPC-100 (Oyo Bunko Kiki K.K.)

Recrystallization from methanol gave 2,6-diethyl-3,5-diphenylpyrazine as colorless needles, m.p. 90-91°C. Physical data of the product support the structure (1) below.

Elementary analysis: ($C_{20}H_{20}N_2$)

Calc'd: C, 83.29%; H, 6.99%; N, 9.80%

Found : C, 83.21%; H, 7.01%; N, 9.80%

MASS (m/z): 288 (molecular ion peak)

$^1$H-NMR ($CDCl_3$) δ (ppm):

1.28(6H,t,J=7.5Hz), 2.90(4H,q,J=7.5Hz), 7.33-7.73(10H,m)

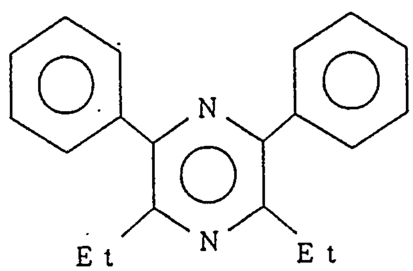

(1)

Example 2

As the second eluate in the column chromatography run in Example 1 was produced 2-ethyl-3,5-diphenylpyrazine (174 mg, 22%).

Recrystallization from methanol gave 2-ethyl-3,5-diphenylpyrazine as colorless needles, m.p. 86-87°C. Physical data of the product support the structure (2) below.

Elementary analysis: ($C_{18}H_{16}N_2$)

Calc'd: C, 83.04%; H, 6.20%; N, 10.76%

Found : C, 83.12%; H, 6.32%; N, 10.79%

MASS (m/z): 260 (molecular ion peak)

$^1$H-NMR ($CDCl_3$) δ (ppm):

1.27(3H,t,J=7.5Hz), 2.91(2H,q,J=7.5Hz), 7.54(8H,m), 8.07(2H,m), 8.90(1H,s)

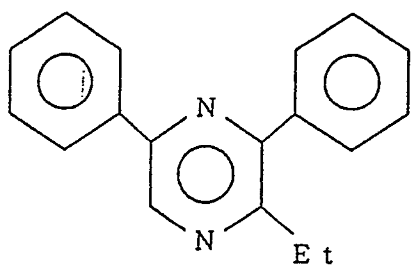

(2)

Example 3

A solution of anisil (2.70 g, 10 mM) and 1,2-diaminocyclohexane (1.14 g, 10 mM) in ethanol (40 ml) was heated under reflux for 2 hours. After cooling, there were precipitated a small amount of yellow needles (anisil). The crystals were separated by suction filtration. The filtrate was removed by distillation under reduced pressure, and to the residue thus obtained was added sulfur (0.64 g). The mixture was heated at 150°C for 30 min. After cooling, the reaction residue was subjected to medium pressure column chromatography on silica gel* (Column: Kieselgel 60, 230-400 mesh, manufactured by Merck, 20 mm x 200 mm; Solvent:

Methylene chloride). There was obtained 1.24 g of 2,3-bis(p-methoxyphenyl)-5,6,7,8-tetrahydroquinoxaline (36%), m.p. 189-190°C (yellow prisms, recrystallized from acetonitrile). Physical data of the product support the structure (3) below.

* UVILOG ALPC-100 (Oyo Bunko Kiki K.K.)

Elementary analysis: ($C_{22}H_{22}N_2O_2$)

Calc'd: C, 76.27%; H, 6.40%; N, 8.09%

Found : C, 75.99%; H, 6.38%; N, 8.13%

MASS (m/z): 346 (molecular ion peak)

$^1$H-NMR ($CDCl_3$) δ (ppm):

1.95(4H,m), 3.00(4H,m), 3.77(6H,s),

6.78(4H,d,J=9Hz), 7.35(4H,d,J=9Hz)

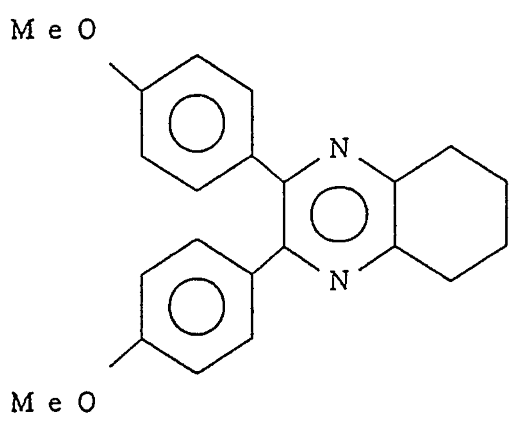

(3)

Example 4

A solution of anisil (10.8 g, 40 mM) and o-phenylenediamine (5.32 g, 40 mM) in methanol was heated

under reflux for 10 hours. After cooling, crystals precipitated were separated by filtration. The crystals were recrystallized from methanol to give 11.0 g of 2,3-bis(p-methoxyphenyl)quinoxaline (80%) as colorless needles, m.p. 151-153°C. Physical data of the product support the structure (4) below.

Elementary analysis: ($C_{22}H_{18}N_2O_2$)

Calc'd: C, 77.17%; H, 5.30%; N, 8.18%

Found : C, 77.03%; H, 5.31%; N, 8.16%

MASS (m/z): 342 (molecular ion peak)

$^1$H-NMR ($CDCl_3$) δ (ppm):

3.78(6H,s), 6.83(4H,d,J=9Hz), 7.46(4H,d,J=9Hz), 7.67(2H,dd,J=6Hz,J=3Hz), 8.08(2H,dd,J=6Hz,J=3Hz)

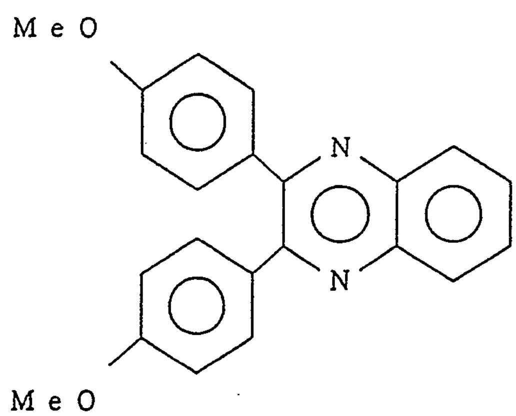

(4)

Example 5

A solution of 2.57 g of 2,3-diphenyl-5,6-dihydropyrazine, 1.40 g of o-chlorobenzaldehyde and 0.672 g

of potassium hydroxide in 20 ml of methanol was allowed to react by heating under reflux for 1 hour. The methanol was distilled off from the reaction mixture under reduced pressure. To the residue was added 50 ml of water followed by extraction with three portions of ethyl acetate. The organic layer of the extract was washed with water and dried over anhydrous sodium sulfate. The solvent was then distilled off under reduced pressure. The residue from the extract was subjected to column chromatography on silica gel. There was produced a crude product from the fraction eluted with hexane-ethyl acetate (8:1). The crude product was recrystallized from a methanol-water mixture to yield 2.633 g of 2,3-diphenyl-5-(o-chlorobenzyl)pyrazine (74%) as colorless prisms, m.p. 112-114°C. Physical properties of the product support the structure (5) below.

Elementary analysis: ($C_{23}H_{17}ClN_2$)

Calc'd: C, 77.41%; H, 4.80%; N, 7.85%

Found : C, 77.13%; H, 4.81%; N, 7.83%

MASS (m/z): 356 (molecular ion peak)

$^1$H-NMR ($CDCl_3$) δ (ppm): 4.42(2H,s), 8.47(1H,s)

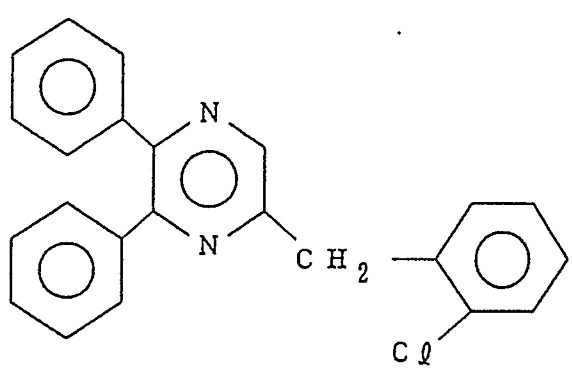

(5)

Example 6

The same procedures as in Example 5 were repeated using 2,3-diphenyl-5,6-dihydropyrazine (2.57 g) and m-chlorobenzaldehyde (1.40 g). There was obtained 3.338 g of 2,3-diphenyl-5-(m-chlorobenzyl)pyrazine (94%) as colorless prisms, m.p. 58-59°C (recrystallized from methanol-water). Physical data of the product support the structure (6) below.

Elementary analysis: ($C_{23}H_{17}ClN_2$)

Calc'd: C, 77.41%; H, 4.80%; N, 7.85%

Found : C, 77.35%; H, 4.75%; N, 7.80%

MASS (m/z): 356 (molecular ion peak)

$^1$H-NMR ($CDCl_3$) δ (ppm): 4.18(2H,s), 8.40(1H,s)

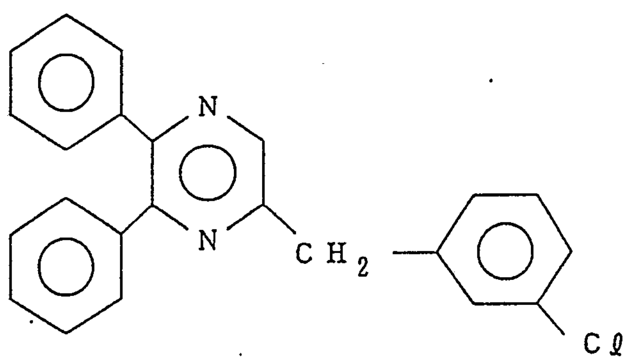

(6)

Example 7

The same procedures as in Example 5 were repeated using 2,3-diphenyl-5,6-dihydropyrazine (2.57 g) and p-chlorobenzaldehyde (1.40 g). There was obtained 3.358 g of 2,3-diphenyl-5-(p-chlorobenzyl)pyrazine (94%) as colorless

prisms, m.p. 105-106°C (recrystallized from isopropyl alcohol). Physical properties of the product support the structure (7) below.

Elementary analysis: ($C_{23}H_{17}ClN_2$)

Calc'd: C, 77.41%; H, 4.80%; N, 7.85%

Found : C, 77.54%; H, 4.80%; N, 7.86%

MASS (m/z): 356 (molecular ion peak)

$^1$H-NMR ($CDCl_3$) δ (ppm): 4.20(2H,s), 8.40(1H,s)

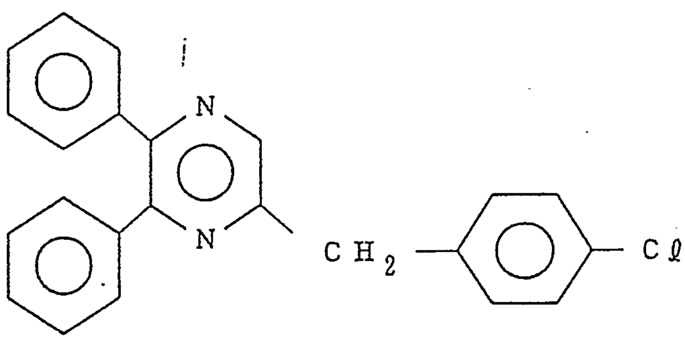 (7)

Example 8

The same procedures as in Example 5 were repeated using 2,3-diphenyl-5,6-dihydropyrazine (2.57 g) and p-bromobenzaldehyde (1.85 g). There was obtained 3.87 g of 2,3-diphenyl-5-(p-bromobenzyl)pyrazine (97%) as colorless needles, m.p. 100-101°C (recrystallized from methanol). Physical data of the product support the structure (8) below.

Elementary analysis: ($C_{23}H_{17}BrN_2$)

Calc'd: C, 68.83%; H, 4.27%; N, 6.98%

Found : C, 68.84%; H, 4.24%; N, 6.97%

MASS (m/z): 401 (molecular ion peak)

$^1$H-NMR ($CDCl_3$) δ (ppm): 4.23(2H,s), 8.48(1H,s)

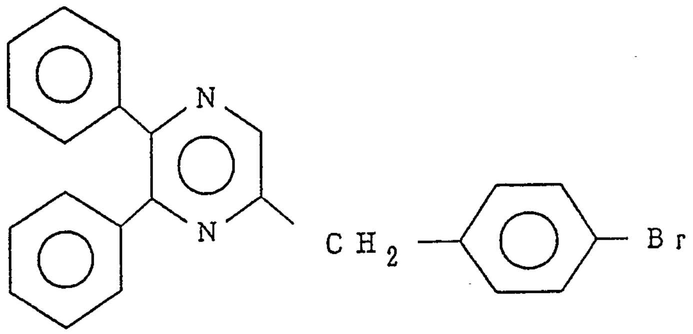

(8)

Example 9

The same procedures as in Example 5 were repeated using 2,3-diphenyl-5,6-dihydropyrazine (2.57 g) and p-dimethylaminobenzaldehyde (1.49 g). There was obtained 3.36 g of 2,3-diphenyl-5-(p-dimethylaminobenzyl)pyrazine (92%) as colorless prisms, m.p. 80-82°C (recrystallized from methanol-water). Physical data of the product support the structure (9) below.

Elementary analysis: ($C_{25}H_{23}N_3$)

Calc'd: C, 82.16%; H, 6.34%; N, 11.50%

Found : C, 82.14%; H, 6.30%; N, 11.50%

MASS (m/z): 365 (molecular ion peak)

$^1$H-NMR ($CDCl_3$) δ (ppm):

2.88(6H,s), 4.12(2H,s), 8.40(1H,s)

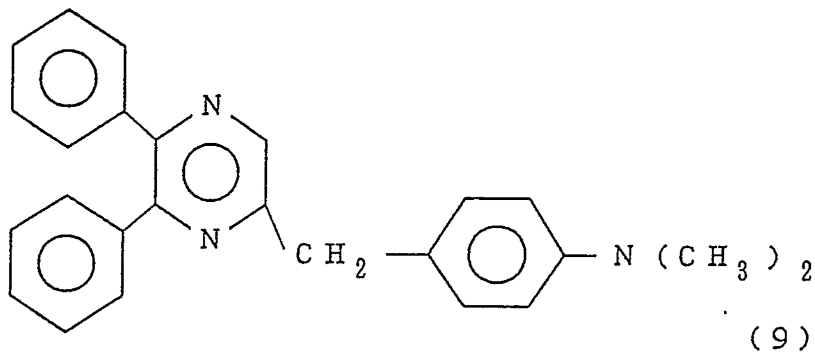

(9)

Example 10

The same procedures as in Example 5 were repeated using 2,3-diphenyl-5,6-dihydropyrazine (2.57 g) and 1-naphthaldehyde (1.56 g). There was obtained 3.30 g of 2,3-diphenyl-5-(1-naphthyl)methylpyrazine (89%) as colorless prisms, m.p. 91-92°C (recrystallized from methanol-water). Physical data of the product support the structure (10) below.

Elementary analysis: ($C_{27}H_{20}N_2$)

Calc'd: C, 87.06%; H, 5.41%; N, 7.52%

Found : C, 86.96%; H, 5.43%; N, 7.50%

MASS (m/z): 372 (molecular ion peak)

$^1$H-NMR ($CDCl_3$) δ (ppm): 4.70(2H,s), 8.30(1H,s)

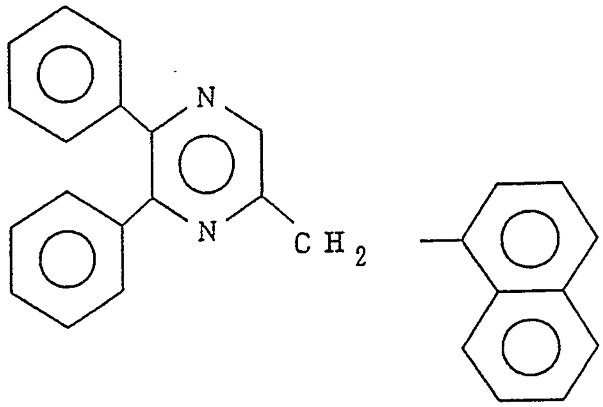

(10)

Example 11

2,3-Bis(p-methoxyphenyl)pyrazine 1,4-dioxide (1.80 g, 5.6 mM) was mixed with phosphorus oxychloride (20 ml), and the mixture was allowed to react by heating under reflux for one hour. After cooling, the reaction mixture was poured onto ice water, subsequently made basic with potassium carbonate and extracted with methylene chloride. After the solvent was distilled off, there was a pale yellow extraction residue. The residue was subjected to column chromatography on silica gel (Wakogel C-200, 32 g; Solvent: A benzene-AcOEt mixture). There was obtained as the first eluate 1.50 g of 2,3-bis(p-methoxyphenyl)-5,6-dichloropyrazine (78%).

Recrystallization from hexane afforded 2,3-bis(p-methoxyphenyl)-5,6-dichloropyrazine as colorless needles, m.p. 120-121°C. Physical data of the product support the

structure (11) below.

Elementary analysis: ($C_{18}H_{14}Cl_2N_2O_2$)

Calc'd: C, 59.85%; H, 3.91%; N, 7.76%

Found : C, 60.02%; H, 3.88%; N, 7.79%

MASS (m/z): 360 (molecular ion peak)

$^1$H-NMR ($CDCl_3$) δ (ppm):

3.77(6H,s), 6.83(4H,d,J=7.5Hz), 7.43(4H,d,J=7.5Hz)

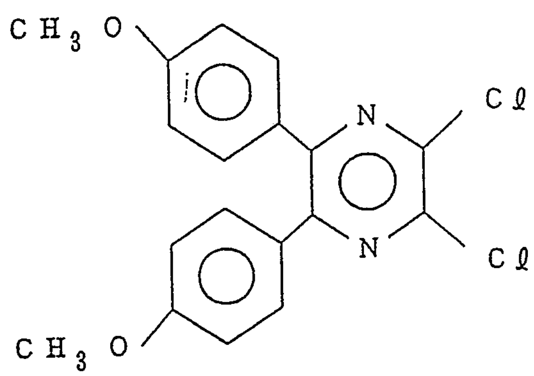

(11)

Example 12

2,3-Bis(p-methoxyphenyl)pyrazine dioxide (3.92 g, 12.7 mM) was mixed with phosphorus oxychloride (20 ml), and the mixture was allowed to react by heating under reflux for 30 min. After cooling, the reaction mixture was poured onto ice water, and subsequently made alkaline with potassium carbonate. There was then precipitated a yellowish orange solid which was recovered by filtration and subjected to column chromatography on silica gel (Wakogel C-200, 70 g;

Solvent: Hexane-methylene chloride=1:1). There was obtained 2.82 g of 2,3-bis(p-methoxyphenyl)-5-chloropyrazine (76%), m.p. 127-128°C (pale yellow prisms, recrystallized from ethanol). Physical data of the product support the structure (12) below.

Elementary analysis: ($C_{18}H_{15}ClN_2O_2$)

Calc'd: C, 66.17%; H, 4.63%; N, 8.57%

Found : C, 65.91%; H, 4.63%; N, 8.60%

MASS (m/z): 326 (molecular ion peak)

$^1$H-NMR ($CDCl_3$) δ (ppm):

3.77(6H,s), 6.77(4H,d,J=7Hz), 7.33(2H,d,J=7Hz), 7.37(2H,d,J=7Hz), 8.42(1H,s)

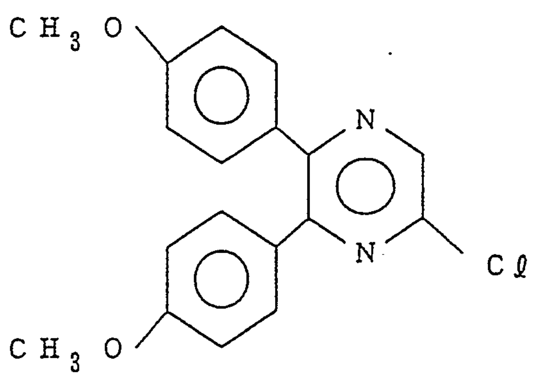

(12)

Example 13

A solution of 2,3-bis(p-methoxyphenyl)-5-chloropyrazine (3.27 g, 10 mM), potassium cyanide (975 mg, 15 mM) and tetrakis(triphenylphosphine)palladium (580 mg, 0.5 mM) in anhydrous DMF (50 ml) was heated under reflux for 4 hours under argone stream. The solvent was removed by distillation under reduced pressure. To the residue was

added water (100 ml), and the mixture was extracted with methylene chloride. The solvent was distilled off to give a dark brown viscous material, which was then subjected to medium pressure column chromatography on silica gel* (Column: Kieselgel 60, 230-400 mesh, manufactured by Merck, 20 mm x 200 mm; Solvent: Hexane-AcOEt=4:1). There was obtained 2.66 g of 2,3-bis(p-methoxyphenyl)-5-cyanopyrazine (84%), m.p. 110-112°C (pale yellow needles, recrystallized from ethanol). Physical data of the product support the structure (13) below.

* UVILOG ALPC-100(Oyo Bunko Kiki K.K.)

Elementary analysis: ($C_{19}H_{15}N_3O_2$)

Calc'd: C, 71.91%; H, 4.76%; N, 13.24%

Found : C, 71.89%; H, 4.63%; N, 13.27%

MASS (m/z): 317 (molecular ion peak)

$^1$H-NMR ($CDCl_3$) δ (ppm):

3.77(6H,s), 6.80(4H,d,J=9Hz), 7.43(2H,d,J=9Hz), 7.45(2H,d,J=9Hz), 8.70(1H,s)

IR(KBr) $cm^{-1}$: 2260 (C≡N)

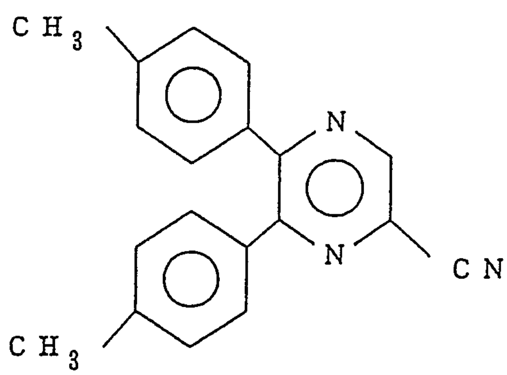

(13)

Example 14

To a solution of 2,3-bis(p-methoxyphenyl)-5-cyanopyrazine (2.0 g, 6.3 mM) in a mixture of methanol (40 ml) and 1,4-dioxane (30 ml) was added a 20% aqueous solution of sodium hydroxide. The mixture was heated under reflux for 4 hours. The solvent was distilled off under reduced pressure. The residue was neutralized with a 5% hydrochloric acid solution followed by extraction with methylene chloride. The solvent was distilled off to give a crude product. The crude product was recrystallized from ethanol to afford 2.07 g of 2,3-bis(p-methoxyphenyl)-pyrazine-5-carboxylic acid (95%) as colorless needles, m.p. 234-235°C. Physical data of the product support the structure (14).

Elementary analysis: ($C_{19}H_{16}N_2O_4$)

Calc'd: C, 67.85%; H, 4.80%; N, 8.33%

Found : C, 67.69%; H, 4.85%; N, 8.34%

MASS (m/z): 336 (molecular ion peak)

IR(KBr) $cm^{-1}$: 1690

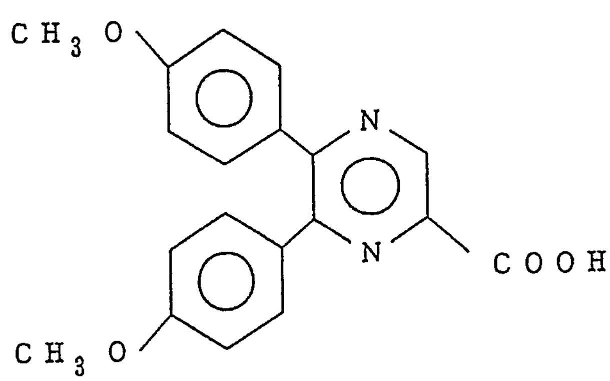

(14)

Example 15

To a solution of 2,3-bis(p-methoxyphenyl)pyrazine-5-carboxylic acid (336 mg, 1mM) in methanol (5 ml) was added concentrated sulfuric acid (0.1 ml). The mixture was heated under reflux for 3 hours. The solvent was distilled off under reduced pressure. To the residue was added water (15 ml) followed by neutralization with potassium carbonate. The resulting mixture was extracted with methylene chloride. The solvent was distilled off to give a crude product. The crude product was recrystallized from methanol to afford 320 mg of 2,3-bis(p-methoxyphenyl)pyrazine-5-carboxylic acid (91%) as colorless needles, m.p. 140-141°C. Physical data of the product support the structure (15) below.

Elementary analysis: ($C_{20}H_{18}N_2O_4$)

Calc'd: C, 68.56%; H, 5.18%; N, 8.00%

Found : C, 68.40%; H, 5.11%; N, 7.93%

MASS (m/z): 350 (molecular ion peak)

$^1$H-NMR ($CDCl_3$) δ (ppm):

3.77(6H,s), 3.97(3H,s), 6.78(4H,d,J=9Hz), 7.43(4H,d,J=9Hz), 9.12(1H,s)

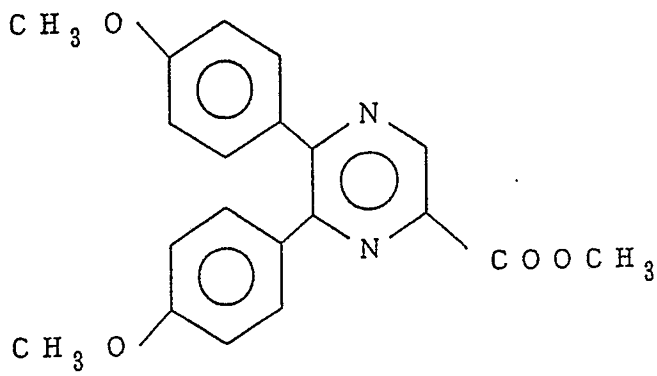

(15)

Pharmacological Test Examples

(Platelet Aggregation-Inhibiting Action)

Blood was drawn from the carotid artery of a rabbit using a syringe containing 1/10 volume of a 3.8% solution of sodium citrate. The blood was centrifuged to obtain platelet-rich plasma (PRP: 5 x $10^5$ cells/µl).

In a cuvette were placed 200 µl of said PRP and 25 µl of a physiological saline solution. The cuvette was set on an aggregometer and warmed at 37°C for 2 min. Into the cuvette was then added 1.25 µl of an ethanol solution of a pyrazine derivative to be tested. After incubated for 3 min., a solution of a platelet-aggregation inducer, arachidonic acid or collagen, was added thereto. Measurements were made for platelet aggregation using an aggregometer (Hematracer IV; Niko Biosciences K.K.). In Table 1 is shown the concentration required for 50% inhibition of the platelet aggregation caused by arachidonic acid (80 µM) or collagen (15 µg/ml). Acetylsalicylic acid

was used as a comparison.

As shown in Table 1, the pyrazine derivatives of the invention were found to possess a marked platelet aggregation-inhibiting activity. The pyrazine derivatives not shown in Table 1 were also found to possess a similar activity. The 50% inhibitory concentration as shown in the table means the concentration of the solution of a pyrazine derivative required for inhibiting the aggregation of platelets by introducing said pyrazine derivative when the platelet aggregation in the absence of any pyrazine derivative of the invention is taken as 100%.

Table 1 Platelet aggregation-inhibiting activity

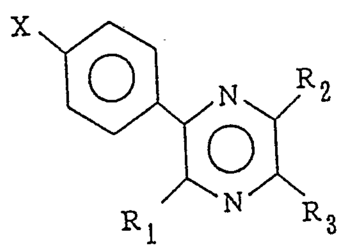

| Substituent | | | | 50% aggregation-inhibitory concentration (mole) | |
|---|---|---|---|---|---|
| X | R1 | R2 | R3 | Arachidonic acid | Collagen |
| H | H | 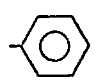 | $C_2H_5$ | $6.0 \times 10^{-6}$ | $1.9 \times 10^{-5}$ |
| H | $C_2H_5$ | 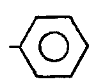 | $C_2H_5$ | $1.7 \times 10^{-6}$ | $3.9 \times 10^{-7}$ |
| $CH_3O$ | 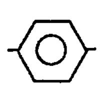$-OCH_3$ | 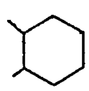 | | $1.5 \times 10^{-8}$ | $2.4 \times 10^{-8}$ |
| $CH_3O$ | 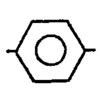$-OCH_3$ | 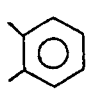 | | $3.8 \times 10^{-8}$ | $9.8 \times 10^{-9}$ |

Table 1 Platelet aggregation-inhibitory concentration (cont'd)

| Substituent | | | | 50% aggregation-inhibitory concentration (mole) | |
|---|---|---|---|---|---|
| X | R1 | R2 | R3 | Arachidonic acid | Collagen |
| H | 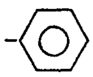 | H | 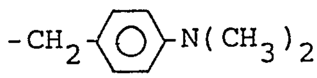 | $4.4 \times 10^{-6}$ | $3.6 \times 10^{-5}$ |
| H | 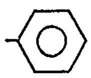 | H | 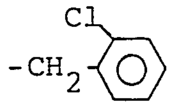 | $4.5 \times 10^{-6}$ | $4.4 \times 10^{-5}$ |
| H | 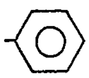 | H | 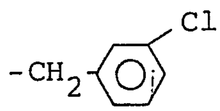 | $8.2 \times 10^{-5}$ | $3.4 \times 10^{-5}$ |
| H | 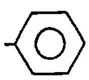 | H | 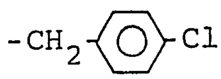 | $1.6 \times 10^{-5}$ | $1.0 \times 10^{-4}$ |
| H | 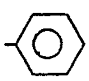 | H | 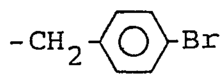 | $8.4 \times 10^{-5}$ | $7.4 \times 10^{-5}$ |
| H | 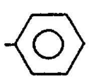 | H | 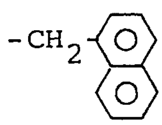 | $4.3 \times 10^{-5}$ | $3.1 \times 10^{-5}$ |

Table 1 Platelet aggregation-inhibitory concentration (cont'd)

| Substituent | | | | 50% aggregation-inhibitory concentration (mole) | |
|---|---|---|---|---|---|
| X | $R_1$ | $R_2$ | $R_3$ | Arachidonic acid | Collagen |
| $-OCH_3$ | 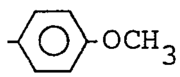 | H | -CN | $2.4 \times 10^{-8}$ | $1.5 \times 10^{-8}$ |
| $-OCH_3$ | 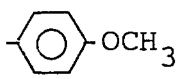 | H | -COOH | $2.6 \times 10^{-7}$ | $5.0 \times 10^{-6}$ |
| $-OCH_3$ | 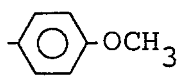 | H | $-COOCH_3$ | $3.0 \times 10^{-9}$ | $1.3 \times 10^{-7}$ |
| $-OCH_3$ | 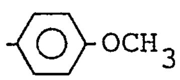 | H | -Cl | $3.8 \times 10^{-9}$ | $9.8 \times 10^{-9}$ |
| $-OCH_3$ | 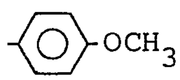 | Cl | -Cl | $1.4 \times 10^{-8}$ | $2.2 \times 10^{-7}$ |
| -CN | 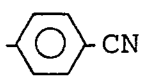 | H | $-CH_3$ | $1.0 \times 10^{-6}$ | $1.0 \times 10^{-6}$ |

〔Cyclooxygenase-inhibiting Activity〕

In a centrifuge tube was collected 9 volumes of blood from the carotid artery of a rabbit per volume of a 3.8% solution of sodium citric acid contained therein using a canula. Platelet-rich plasma was obtained by centrifugation. To the platelet-rich plasma was added a 77 mM solution of EDTA in a volume of 1/10 per volume of said plasma. After mixing thoroughly, the mixture was subjected to centrifugation at 2500 r.p.m. at room temperature for 10 min. The supernatant was discarded, and the platelets were suspended in approximately 3 ml of a wash (134 mM of sodium chloride, 15 mM of trisaminomethane, 1 mM of EDTA and 5 mM of D-glucose dissolved in distilled water with a pH adjusted to 7.4 with 1N-hydrogen chloride), and the suspension was centrifuged at 2000 rpm at room temperature for 10 min. The supernatant was discarded, and the precipitated platelets were suspended in a 1/15 M phosphate buffer solution at pH 8.0 to adjust the number of the platelets to 6-8 x $10^5$ cells/μl.

The washed platelets thus produced were used as an enzyme source for cyclooxygenase.

In a test tube with a ground stopper were placed 3 μg of arachidonic acid and 0.2 μCi (1 μg) of $^{14}C$-labeled arachidonic acid (in toluene solution), to which was added one drop of a propylene glycol/ethanol mixture (1:3 in volume). The toluene and the ethanol were evaporated under nitrogen gas stream. To the test tube was added 50 μl of a

test solution and then 500 µl of the washed platelets followed by reaction at 37°C for 3 min.

The reaction mixture was adjusted to pH 2-3 by the addition of 3 drops of 0.5 N-hydrogen chloride while cooling with ice. To the resulting mixture was added 2 ml of ethyl acetate, and extraction was made by shaking for 10 min. followed by centrifugation at 2500 rpm at 4°C for 10 min.

The supernatant was transferred to a flask and concentrated. The residue was dissolved in 100 µl of ethanol, and the entire solution was spotted on a thin layer plate of silica gel (60F 254 manufactured by Merck).

Development was made with a developing solvent (chloroform/methanol/acetic acid/water = 90:8:1:0.8) by approximately 18 cm, and then the inhibiting activity was determined by measuring sum of the radioactivities of prostaglandin $E_2$, prostaglandin $D_2$ and HHT by a radiochromatogram scanner. The result is shown in Table 2. The pyrazine derivatives of the invention not shown in Table 2 were also found to possess a similar activity.

Table 2 Cyclooxygenase-inhibiting activity

| Substituent | | | | 50% inhibitory concentration |
|---|---|---|---|---|
| X | R1 | R2 | R3 | (mole) |
| $-OCH_3$ | 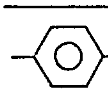$OCH_3$ | H | -CN | $5 \times 10^{-6}$ |

〔Acute Toxicity〕

An acute toxicity test was conducted in male ICR

mice (5 week-old) by oral administration. $LD_{50}$ values were 300 mg/kg or higher for all of the pyrazine derivatives of the invention tested thereby demonstrating high safety.

(Industrial Applicability)

According to the invention there are provided novel pyrazine derivatives and pharmaceutical preparations containing the same.

As said compounds of the invention remarkably inhibit the platelet aggregation induced by arachidonic acid or collagen, they can be used as a preventive agent for diseases caused by platelet aggregation, particularly for thromboses in which platelet aggregation participates such as myocardial infarction, ischemic attack after cerebral hemorrhage and cerebral infarct.

The above-mentioned compounds of the invention also have a cyclooxygenase-inhibiting activity and can be used as an antiinflammatory agent.

Therefore, the present invention is utilizable in a field of pharmaceutical industries.

Claim

1) A pyrazine derivative represented by the general formula (I)

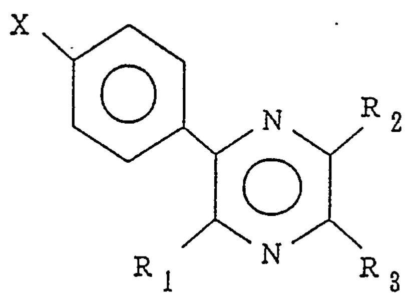

(I)

wherein X represents a hydrogen atom, a halogen atom, a lower alkyl group, a lower alkoxy group or a cyano group; $R_1$ represents a hydrogen atom, a lower alkyl group or a group having the formula 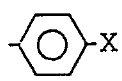 in which X has the same meaning as defined above; $R_2$ represents a hydrogen atom, a halogen atom or a group having the formula 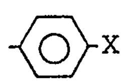 in which X has the same meaning as defined above; $R_3$ represents a hydrogen atom, a halogen atom, a lower alkyl group, a cyano group, a naphthylmethyl group, a benzyl group having the formula 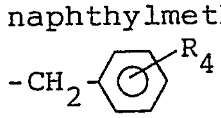 in which $R_4$ represents a hydrogen atom, a halogen atom, or a lower alkylamino group, a carboxyl group or a lower alkoxycarbonyl group, or $R_2$ and $R_3$ together with the carbon atoms to which they are connected represent a cyclohexane ring or a benzene ring except for the case where X is a hydrogen atom, $R_1$ is a group having the formula

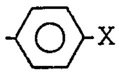X in which X is a hydrogen atom and $R_2$ and $R_3$ independently are a hydrogen atom or X is a hydrogen atom, $R_1$ is a hydrogen atom, $R_2$ is a group having the formula 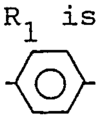X in which X is a hydrogen atom and $R_3$ is a methyl group.

2) A pyrazine derivative according to Claim 1 represented by the general formula (II)

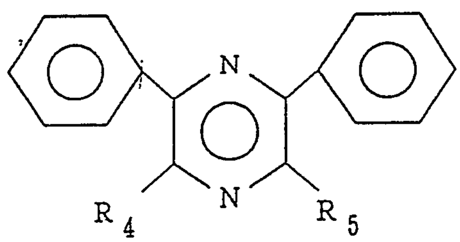

(II)

wherein $R_4$ and $R_5$ which may be the same or different independently represent a hydrogen atom or a lower alkyl group except for the case where $R_4$ represents a hydrogen atom and $R_5$ represents a methyl group.

3) A pyrazine derivative according to Claim 1 represented by the general formula (III) or (IV)

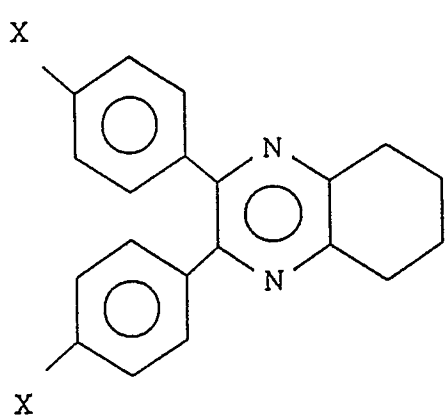

(III)

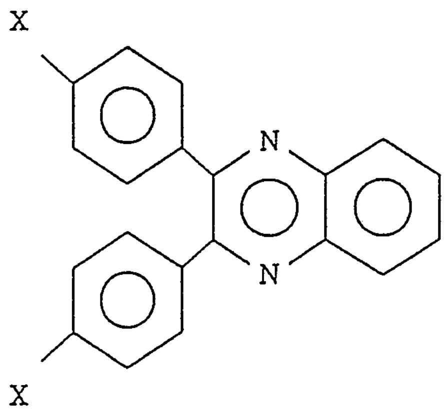

(IV)

wherein X represents a hydrogen atom, a halogen atom, a lower alkyl group, a lower alkoxy group or a cyano group.

4) A pyrazine derivative according to Claim 1 represented by the general formula (V)

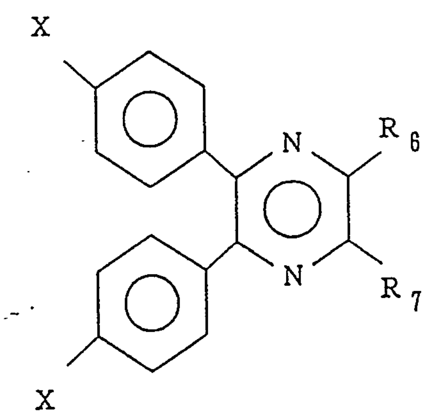

(V)

wherein X represents a hydrogen atom, a cyano group or a lower alkoxy group, $R_6$ represents a hydrogen atom or a halogen atom and $R_7$ represents a hydrogen atom, a halogen atom, a lower alkyl group, a cyano group, a naphthylmethyl group, a benzyl group which may contain as the substituent a halogen atom or a lower alkylamino group, a carboxyl group or a lower alkyloxycarbonyl group.

5) A platelet aggregation-inhibiting agent comprising the pyrazine derivative according to Claim 1.

6) An antiinflammatory agent comprising the pyrazine derivative according to Claim 1.

7) A method for preventing thrombosis which comprises administering an effective amount of the pyrazine derivative according to Claim 1 to patients possibly afflicted with said disease.

8) A method for treating inflammation which comprises administering an effective amount of the pyrazine derivative according to Claim 1 to patients afflicted with said symptom.

9) Use of the pyrazine derivative according to Claim 1 for the preparation of a platelet aggregation-inhibiting agent.

10) Use of the pyrazine derivative according to Claim 1 for the preparation of an antiinflammatory agent.

# INTERNATIONAL SEARCH REPORT

International Application No PCT/JP 88/01141

**I. CLASSIFICATION OF SUBJECT MATTER** (if several classification symbols apply, indicate all) [6]

According to International Patent Classification (IPC) or to both National Classification and IPC

Int.Cl$^4$ C07D241/12, 241/16, 241/24, 241/42, A61K31/495

**II. FIELDS SEARCHED**

Minimum Documentation Searched [7]

| Classification System | Classification Symbols |
|---|---|
| IPC | C07D241/10-241/16, 241/24, 241/36-241/42, A61K/495 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, B1, 46-12726 (Société National de Pétrole d'Aquitaine) 1 April 1971 (01. 04. 71) (Family: none) | 1 - 2 |
| X | JP, A, 49-117480 (Koei Chemical Co., Ltd.) 9 November 1974 (09. 11. 74) (Family: none) | 1 |
| X | JP, A, 58-43961 (Ube Industries, Ltd.) 14 March 1983 (14. 03. 83) (Family: none) | 1, 5-10 |
| X | JP, A, 61-129171 (Ube Industries, Ltd.) 17 June 1986 (17. 06. 86) (Family: none) | 1 |
| X | JP, A, 61-212522 (Terumo Corporation) 20 September 1986 (20. 09. 86) (Family: none) | 1, 4-10 |
| X | JP, A, 61-257978 (Suntory Ltd.) 15 November 1986 (15. 11. 86) &US, A, 4755514 &EP, A, 204172 | 1, 5-10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 1, 1989 (01. 02. 89) | February 13, 1989 (13. 02. 89) |
| **International Searching Authority** | **Signature of Authorized Officer** |
| Japanese Patent Office | |

International Application No. PCT/JP 88/01141

**FURTHER INFORMATION CONTINUED FROM THE SECOND SHEET**

| | | |
|---|---|---|
| X | JP, A, 62-5970 (Terumo Corporation) 12 January 1987 (12. 01. 87) &EP, A, 194686 | 1, 4-10 |
| X | JP, A, 62-89669 (Ube Industries, Ltd.) 24 April 1987 (24. 04. 87) (Family: none) | 1 |
| X | JP, A, 62-234072 (Ube Industries, Ltd.) 14, October 1987 (14. 10. 87) (Family: none) | 1 |
| P | JP, A, 62-270564 (Terumo Corporation) 24 November 1987 (24. 11. 87) &EP, A, 194686 | 1, 4-10 |

**V. ☐ OBSERVATIONS WHERE CERTAIN CLAIMS WERE FOUND UNSEARCHABLE [1]**

This international search report has not been established in respect of certain claims under Article 17(2) (a) for the following reasons:

1. ☐ Claim numbers ............, because they relate to subject matter not required to be searched by this Authority, namely:

2. ☐ Claim numbers ............, because they relate to parts of the international application that do not comply with the prescribed requirements to such an extent that no meaningful international search can be carried out, specifically:

3. ☐ Claim numbers ............, because they are dependent claims and are not drafted in accordance with the second and third sentences of PCT Rule 6.4(a).

**VI. ☐ OBSERVATIONS WHERE UNITY OF INVENTION IS LACKING [2]**

This International Searching Authority found multiple inventions in this international application as follows:

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

FURTHER INFORMATION CONTINUED FROM THE SECOND SHEET

| | | |
|---|---|---|
| P | JP, A, 63-267765 (Ube Industries, Ltd.) 4 November 1988 (04. 11. 88) (Family: none) | 1 |
| X | JP, A, 49-124004 (Tamura Yukimitsu) 27 November 1974 (27. 11. 74) (Family: none) | 1 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers ............, because they relate to subject matter not required to be searched by this Authority, namely:

2.☐ Claim numbers ............, because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

International Application No. PCT/JP 88/01141

**FURTHER INFORMATION CONTINUED FROM THE SECOND SHEET**

| | | |
|---|---|---|
| P | JP, A, 63-10768 (Terumo Corporation) 18 January 1988 (18. 01. 88) (Family: none) | 1, 4-10 |
| P | JP, A, 63-91377 (Ube Industries, Ltd.) 22 April 1988 (22. 04. 88) (Family: none) | 1 |
| X | JP, A, 59-144770 (Ube Industries, Ltd.) 18 August 1984 (18. 08. 84) (Family: none) | 1 |
| P | JP, A, 63-227551 (Ube Industries, Ltd.) 21 September 1988 (21. 09. 88) (Family: none) | 1 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers ..... ......, because they relate to subject matter not required to be searched by this Authority, namely:

2.☐ Claim numbers ............, because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.